# EUROPEAN PATENT APPLICATION

(11) **EP 1 055 735 A2**
(43) Date of publication of application: **29.11.2000**
(21) Application number: 00110932.1
(22) Date of filing: 25.05.2000
(51) Int. Cl.: C12Q 1/68

(54) **Method for gene analysis by hybridisation in the presence of a double-stranded DNA-binding protein**

(30) Priority: 25.05.1999 JP 14474999
(71) Applicant: MITSUBISHI CHEMICAL CORPORATION, Chiyoda-ku, Tokyo 100-0005 (JP)
(72) Inventor: Hatakeyama, Kazuhisa, Mitsubishi Chemical Corp., Inashiki, Ibaraki (JP)
(74) Representative: VOSSIUS & PARTNER

(57) **Abstract**

In a method for gene analysis comprising the step of detecting hybridization between a probe nucleic acid and a sample nucleic acid containing a target sequence that has a sequence complementary to that of the probe nucleic acid, the hybridization is caused on a substrate on which either the probe nucleic acid or the sapmle nucleic acid is immobilized, in the presence of a double-stranded DNA-binding protein to improve analysis speed of a method for gene analysis by hybridization using a probe nucleic acid.

SEQUENCE LISTING
<110> Mitsubishi Chemical Corporation

<120> Method for Gene Analysis

<150> JP 11-144749
<151> 1999-05-25

<160> 9

<210> 1
<211> 15
<212> DNA
<213> Artificial Sequence

<220>
<223> Description of Artificial Sequence : probe for
hybridization

<220>
<221> misc_feature
<222> (1)
<223> n=5' amino-modified C6

<400> 1
natgtaactc gcctt                                                    15

<210> 2
<211> 15
<212> DNA
<213> Artificial Sequence

<220>
<223> Description of Artificial Sequence : probe for
hybridization

<220>
<221> misc_feature
<222> (1)

<223> n=5′ amino-modified C6

<400> 2
natgtaaccc gcctt                                                    15

<210> 3
<211> 28
<212> DNA
<213> Artificial Sequence

<220>
<223> Description of Artificial Sequence : sample for
hybridization

<400> 3
ccaacgatca aggcgagtta catgatcc                                      28

<210> 4
<211> 14
<212> DNA
<213> Artificial Sequence

<220>
<223> Description of Artificial Sequence : probe for
hybridization

<220>
<221> misc_feature
<222> (14)
<223> n=3′ amino-modified C6

<400> 4
atcgcccgga ctcn                                                     14

<210> 5
<211> 14
<212> DNA
<213> Artificial Sequence

<220>
<223> Description of Artificial Sequence : probe for

hybridization

<220>
<221> misc_feature
<222> (14)
<223> n=3' amino-modified C6

<400> 5
atcgcctgga ctcn                                                    14

<210> 6
<211> 20
<212> DNA
<213> Artificial Sequence

<220>
<223> Description of Artificial Sequence : probe for
hybridization

<220>
<221> misc_feature
<222> (1)-(4), (16)-(19)
<223> n=i

<220>
<221> misc_feature
<222> (20)
<223> n=3' amino-modified C6

<400> 6
nnnntcgccc ggactnnnnn                                              20

<210> 7
<211> 20
<212> DNA
<213> Artificial Sequence

<220>
<223> Description of Artificial Sequence : probe for
hybridization

<220>
<221> misc_feature
<222> (1)-(4), (16)-(19)
<223> n=i

<220>
<221> misc_feature
<222> (20)
<223> n=3′ amino-modified C6

<400> 7
nnnntcgcct ggactnnnnn                                                20

<210> 8
<211> 26
<212> DNA
<213> Artificial Sequence

<220>
<223> Description of Artificial Sequence : sample for
hybridization

<400> 8
agtctggag tccgggcgat ggccac                                          26

<210> 9
<211> 63
<212> PRT
<213> Sulfolobus Solfataricus

<300>
<301> Herbert Baumann, Stefan Knapp, Thomas Lundback, Rudolf
Ladenstein and Torleif Hard
<302> Solution structure and DNA-binding properties of a
thermostable protein from the archaeon Sulfobus Solfataricus
<303> Nature structural biology
<304> 1
<305> 11
<306> 808-819
<307> 1994-11-1

<400> 9
Ala Thr Val Lys Phe Lys Tyr Lys Gly Glu Glu Lys Gln Val Asp Ile
1               5                   10                  15
Ser Lys Ile Lys Lys Val Trp Arg Val Gly Lys Met Ile Ser Phe Thr
            20                  25                  30
Tyr Asp Glu Gly Gly Gly Lys Thr Gly Arg Gly Ala Val Ser Glu Lys
        35                  40                  45
Asp Ala Pro Lys Glu Leu Leu Gln Met Leu Glu Lys Gln Lys Lys
    50                  55                  60

## Description

### Field of the Invention

The present invention relates to a method for gene analysis by hybridization, and more specifically, it relates to a novel method for gene analysis preferably utilized for gene analysis by hybridization using a DNA chip and the like, which method can be used for the analysis of nucleic acids, such as nucleotide sequence determination of nucleic acids, gene diagnosis of infectious diseases or genetic diseases, and monitoring of genome DNA expression.

### Description of the Related Art

The analysis of genes by a hybridization technique utilizing a substrate (DNA chip or DNA array) having immobilized probe nucleic acids is widely utilized, for example, for nucleotide sequence determination, gene diagnosis of infectious and genetic diseases, monitoring expression of genome gene and so forth. For instance, SBH [Sequencing By Hybridization, R. Drmanac et al., Science, 260, 1649 (1993)], i.e., nucleotide sequencing via hybridization, is expected to be put into practical use as a high-speed and low cost method. Further, the method for detecting mutations contained in genes using DNA chips [J.G. Hacia et al., Nature Genetics, 14,411-447(1996)] and the method of monitoring gene expression patterns using DNA chips [M. Schena et al., Science, 270,467-470(1995)] are drawing attention as methods enabling quick analysis of significant amounts of gene expression.

For these analyses of genes, an article called DNA chip or DNA array (DNA microarray or DNA macroarray) is utilized, which comprises nucleic acids such as DNA and/or RNA immobilized on a substrate.

As a substrate on which DNA is immobilized, membranes made of resin such as nylon membranes and polypropylene membranes, nitrocellulose membranes, glass plates or silicon plates are utilized. When detection of hybridization is performed by not using radioisotopes, but using, for example, fluorescent substances, it is preferable to utilize glass plates or silicon plates which contain no fluorescent substance.

However, when hybridization is performed on a DNA chip or DNA array (DNA microarray or DNA macroarray), the hybridization velocity is the major factor affecting the speed of gene analysis. The hybridization time required for gene analysis utilizing usual DNA chips or DNA arrays (DNA microarrays or DNA macroarrays) is 1 to 5 hours for gene sequence analysis [J.G. Hacia et al., Nature Genetics, 14, 441-447 (1996)], or 6 to 12 hours for gene sequence analysis [M. Shena et al., Proc. Natl. Acad. Sci. USA, Vol. 93, pp. 10614-10619 (1996)], and this has been a major problem in realizing gene analysis of higher speed utilizing the aforementioned method.

### Summary of the Invention

The object of the present invention is to increase the speed of gene analysis by hybridization utilizing a probe nucleic acid.

According to the present invention it was found that gene analysis speed can be increased by performing hybridization on a DNA chip or DNA array (DNA microarray or DNA macroarray), which consists of a substrate having an immobilized probe nucleic acid, in the presence of a double-stranded DNA-binding protein derived from a thermophilic bacterium. That is, it is considered that a double-stranded DNA-binding protein was bound to a double-stranded DNA in a DNA hybridization system in an equilibrated state to forward the reaction in a single direction (complementary double-stranded DNA forming direction), and that the heat resistance of the protein enabled the reaction at high temperatures and thereby realized higher speed and high-throughput of the gene analysis. The present invention has been accomplished on the basis of these findings.

That is, the present invention provides a method for gene analysis comprising the step of detecting hybridization between a probe nucleic acid and a sample nucleic acid containing a target sequence that has a sequence complementary to that of the probe nucleic acid, wherein either the probe nucleic acid or the sample nucleic acid is immobilized on a substrate, at least one of the probe nucleic acid and the sample nucleic acid is DNA, and the hybridization is caused in the presence of a double-stranded DNA-binding protein.

The present invention also provides the aforementioned method for gene analysis wherein the sample nucleic acid is DNA.

The present invention further provides the aforementioned method for gene analysis wherein the double-stranded DNA-binding protein is derived from a hyperthermophilic bacterium.

The present invention further provides the aforementioned method for gene analysis wherein the double-stranded DNA-binding protein is derived from an archaebacterium.

The present invention further provides the aforementioned method for gene analysis wherein the double-stranded DNA-binding protein is derived from a bacterium belonging to the genus *Sulfolobus*.

The present invention further provides the aforementioned method for gene analysis wherein the double-stranded DNA-binding protein is derived from *Sulfolobus solfataricus*.

The present invention further provides the aforementioned method for gene analysis wherein the double-stranded DNA-binding protein is Sso7d protein derived from *Sulfolobus solfataricus.*

The present invention further provides the aforementioned method for gene analysis wherein the double-stranded DNA-binding protein is a protein having homology of 75% or more to the protein represented by the amino acid sequence of SEQ ID NO: 9.

The present invention further provides the aforementioned method for gene analysis wherein the sample nucleic acid is labeled.

The present invention further provides the aforementioned method for gene analysis wherein amount of the sample nucleic acid containing the target sequence is analyzed based on intensity of hybridization signal.

The present invention further provides the aforementioned method for gene analysis wherein detecting hybridization is performed by using a plurality of probe nucleic acids and then polymorphism in the target sequence is detected based on the result of detection of hybridization.

The present invention further provides the aforementioned method for gene analysis wherein detecting hybridization is performed by using a plurality of probe nucleic acids and then nucleotide sequence of the sample nucleic acid is determined based on the result of detection of hybridization.

The present invention further provides a test kit for detection of hybridization between a probe nucleic acid and a sample nucleic acid containing a target sequence that has a sequence complementary to that of the probe nucleic acid, which comprises at least a double-stranded DNA-binding protein.

### DETAILED DESCRIPTION OF THE PREFERRED EMBODIMENTS

The present invention will be explained in detail hereafter.

In the present invention, the term "double-staranded DNA-binding protein" refers to a protein which binds to chromosome of eucaryote or that of prokaryote strongly and concerns retention of higher-order structure of chromosome. That is, it comprises a protein having function to stabilize a complementary double-staranded DNA.

In the present invention, the term "sample nucleic acid" refers to a nucleic acid which is a subject of analysis such as nucleotide sequence determination or expression analysis, and it may be either DNA or RNA.

In the present invention, the term "probe nucleic acid" refers to a nucleic acid which is utilized for detection of a target gene in a sample nucleic acid, and which may be either DNA or RNA. Examples of the probe nucleic acid include a probe containing an oligonucleotide comprising usual base of nucleic acid that is A (adenine), T (thymine), G (guanine), C (cytosine) and U(uracil), and a probe consisting of a DNA fragment amplified by PCR and having a length of approximately 50-2,000 nucleotides. The length of the probe nucleic acid is not particularly limited so long as it is a length hybridizable with the sample nucleic acid. Those having a length of 6-90 nucleotides, preferably 8-30 nucleotides, are usually used. However, nucleic acids of either longer or shorter than these lengths may also be used.

As the probe nucleic acid, there may further be used a probe nucleic acid containing an oligonucleotide which comprises a modified nucleotide, for example, hypoxanthines such as inosine (Japanese Patent Laid-open (Kokai) No. 8-70900(U.S. Patent No. 5,738,993)), 5-nitroindole, 3-nitropyrrole (Japanese Patent Laid-open (Kokai) No. 10-262675), 2-aminoadenine, 5-(1-propynyl)uracil (Tetrahedron Letters, Vol.33, p. 5307-5310) and so forth.

In the probe nucleic acid, as described in Japanese Patent Laid-open (Kokai) No. 8-70900 (U.S. Patent No. 5,738,993), a non-specific region may be ligated to at least one of the ends of the sequence region substantially complementary to the target sequence in the sample nucleic acid. Ligation of such a non-specific region enables increase of hybridization sensitivity as well as facilitates differentiation between a complementary hybrid and a hybrid having a mismatch, in particular, end mismatch.

The non-specific region consists of at least one nucleotide which has a base that can form a base pair with a nucleotide constituting normal nucleic acids, but is different from those of the nucleotides constituting normal nucleic acids, or an oligomer thereof. Such a base is preferably one that can associate with any of bases constituting normal nucleic acid equally, and strength of such association is preferably weaker than that of the base pairs constituting a specific pairing. As a specific example of such a base, hypoxanthine, 5-nitroindole, 3-nitropyrrole and so forth can be mentioned. As a specific example of such a nucleotide, there can be mentioned deoxyinosine, which is a deoxyribonucleotide having hypoxanthine as a base.

The number of nucleotides or oligomers thereof constituting the non-specific region is not particularly limited. However, it is preferably 2-20, more preferably 2-8. The location for ligation of the non-specific region to the specific region is not particularly limited as well, and it may be either of the 5' end and 3' end of the specific region. Both of the 5' end and 3' end of the specific region may be each ligated with a non-specific region. Among these cases, the latter case is particularly preferred. While the ratio of lengths of the specific region and the non-specific region may vary depending on the length of the specific region or the GC content, the length of the specific region is preferably equal to or longer than that of the non-specific region.

By providing a non-specific region in the probe nucleic acid as described above, the gene analysis can be performed with higher precision.

The aforementioned probe nucleic acid and the sample nucleic acid can easily be synthesized by using a commercially available DNA synthesizer.

The probe nucleic acid is utilized to detect a sample nucleic acid comprising a nucleotide sequence complementary to that of the probe nucleic acid. This complementary sequence to the probe nucleic acid is referred to as a "target sequence" in the present invention.

The method of the present invention is characterized in that the hybridization between a probe nucleic acid and a sample nucleic acid is performed in the presence of a double-stranded DNA-binding protein. The hybridization may be performed in the same manner as conventional methods for gene analysis by hybridization, except for the use of the double-stranded DNA-binding protein. In addition, in the method of the present invention, at least one of the aforementioned probe nucleic acid or the sample nucleic acid should be DNA.

In the method of the present invention, it is preferable that the probe nucleic acid or the sample nucleic acid should be immobilized on a substrate, and the hybridization between the probe nucleic acid and a sample nucleic acid should be performed on the substrate. In this case, it is preferable that the sample nucleic acid should be labeled to detect whether or not the hybridization has occurred. The method used for labeling is not particularly limited, and for example, it may include methods utilizing radioisotopes, fluorescent dyes, biotin and so forth.

As a substrate for hybridization, various kinds of materials, for example, membranes made of resin such as nylon membranes and nitrocellulose membranes, glass, silicon, etc are usually used. However, in the present invention, the substrate is not limited to these materials, and any substrate can be utilized so long as DNA and RNA can be immobilized on it in some way.

In this specification, the substrate on which a probe nucleic acid is immobilized is also referred to as a "DNA chip" or a "DNA array (DNA microarray or DNA macroarray)" for convenience. However, as was described earlier, this does not mean that the nucleic acid to be immobilized must be DNA, as it may also be RNA. Those comprising membranes such as nylon membrane, nitrocellulose membrane and so forth having an immobilized probe nucleic acid are generally referred to as "DNA (macro)array", and those comprising a rigid substrate composed of glass, silicon etc. having an immobilized probe nucleic acid are sometimes referred to as "DNA chip" or "DNA (micro)array". In the present invention, all of these may be used.

As the method for immobilization of the probe nucleic acid or the sample nucleic acid onto the substrate, there can be mentioned the method utilizing synthesis of nucleic acids directly onto a substrate [A.C. Pease et al., Proc. Natl. Acad. Sci. USA, 91, 5022-5026 (1994)], the methods comprising immobilization of synthesized nucleic acids [Z. Guo et al., Nucl. Acids Res., 22, 5456-5465 (1994)] or PCR products [M. Shena et al., Proc. Natl. Acad. Sci. USA, vol. 93, pp. 10614-10619 (1996)] and so forth onto a substrate.

For example, the synthesis of a probe nucleic acid or a sample nucleic acid can be performed according to a standard protocol. In the synthesis, it is preferable to synthesize them with a cycle in which trityl groups, which are usually used as a protective group for the 5' end, are not removed, when the following purification method is used. The synthesized nucleic acid is preferably purified by using Poros Oligo R3 (produced by PerSeptive Biosystems) or the like.

It is preferable to adjust the concentrations of the probe nucleic acid or the sample nucleic acid to predetermined concentrations prior to immobilization onto the substrate or hybridization thereof. For example, these nucleic acids are concentrated to dryness, and then suspended in a 0.5 M sodium hydrogencarbonate buffer (pH 8.4), TE buffer or the like, and they are quantified based on absorbance at 260 nm and adjusted to 1 nmol/µl.

Nucleic acids can be immobilized onto the substrate, for example, by bonding amino groups of amino-modified oligonucleotides to a nylon membrane having anionic carboxyl groups on its surface at a high density through amide bonds, as described below.

As for the substrate, nitrocellulose membranes, glass and so forth can also be utilized. It is particularly preferable to utilize glass when the sample DNA will be labeled with a fluorescent substance.

As the method for labeling and detection of nucleic acid, the RI method utilizing [γ-³²P] ATP was used in the example described later, but either the fluorescent method or the biotin-avidin method may be used as well. Examples of the fluorescent substance include Cy3, Cy5, FITC (fluorescein isothiocyanate) and so forth. As for the labeling method, in addition to the 5' end labeling method, the random primer labeling method, the nick translation method and so forth can be utilized as well.

Methods of the aforementioned synthesis of nucleic acid, hybridization, the labeling of nucleic acids and so forth are described in references well known to those skilled in the art, for example, Maniatis, T. et al., "Molecular Cloning, A Laboratory Manual, Second Edition", Cold Spring Harbor Laboratory Press (1989)) and so forth.

Although various kinds of double-stranded DNA-binding proteins are known as double-stranded DNA-binding proteins, it is preferable to utilize a thermostable double-stranded DNA-binding protein which is derived from a hyperthermophilic bacterium (bacterium which can grow at temperatures of 90°C or higher). Hyperthermophilic bacteria are assumed to have some double-stranded DNA-binding protein because, when double-stranded DNA is separated from genome of hyperthermophilic bacteria and heated to 100°C, the DNA becomes single-stranded DNA.

Further, among hyperthermophilic bacteria, archaebacteria such as *Methanobacterium, Methanococcus, Archaeglobus, Pyrococcus* and so forth contain double-stranded DNA-binding proteins such as histone-like proteins and HU protein.

Other than those, as double-stranded DNA-binding proteins derived from archaebacteria, there have been known proteins derived from *Sulfolobus* bacteria such as Sso7d protein derived from *Sulfolobus solfataricus* [A. Guagliardi et al., J. Mol. Biol., Vol. 267, p.841-848 (1997)] ,Sac7d and Sac7e proteins derived from *Sulfolobus acidocaldarius* [J.G. McAfee et al., Biochemistry, Vol. 34, p. 10063-10077 (1955)], Sac7a and Sac7b proteins derived from *Sulfolobus acidocaldarius* [Kimura, M. et al., FEBS Letts., Vol. 176, p. 176-178(1984); Choli, T. et al., J. Biol. Chem., Vol.263, p.7087-7093(1998)] and so forth. However, it is more preferable to utilize the Sso7d protein derived from *Sulfolobus solfataricus*.

As shown in SEQ ID NO: 9, the Sso7d protein is a protein consisting of 63 amino acids [H. Baumann et al., Nature Structural Biology, Vol. 1, p. 808-819 (1994)]. In the present invention, a protein consisting of the 63 amino acids or a protein having a homology thereto of 75% or more in amino acid sequence are particularly preferably used as the double-stranded DNA-binding protein.

In the present invention, analysis of the homology is carried out by using the Lipman-Pearson method.

As software for analysis, "Genetyx" produced by Software Development Co., LTD. is used.

The result of analysis by using the method is shown as follows.
Sso7d vs. Sac7a : 84.5%
Sso7d vs. Sac7b : 87.5%
Sso7d vs. Sac7d : 84.5%
Sso7d vs. Sac7e : 83.3%

These double-stranded DNA-binding proteins have a function of stabilizing the double strands complementarily hybridized, and it is considered that they can maintain the double strands acceleratedly hybridized at high temperatures as it is without causing re-dissociation thereof. It is considered that a protein having such a function can be utilized in the present invention like the specifically aforementioned protein. Any special conditions are not particularly required for the expression of the aforementioned function of the double-stranded DNA-binding protein, and ordinary hybridization conditions with the presence of dithiothreitol(DTT), magnesium ions and so forth are sufficient.

Further, the double-stranded DNA-binding protein is preferably purified. The purification method may be a conventional purification method for proteins, and for example, purification can be performed by the method of Bauman (Nature Structural Biology, Vol. 1, p.808-819 (1994)) and so forth.

Specifically, the purification of the Sso7d protein, for example, can be performed by the following procedure. First, the *Sulfolobus solfataricus* strain DSM 1618 (strain IFO 15331) is cultured, and then the obtained bacterial cells are disrupted by a French press or the like and centrifuged. The obtained supernatant fraction is fractionated by using a MonoQ (produced by Pharmacia) column, and the target fraction is concentrated. The fraction is fractionated by using a Superose 6 column, and the target fraction is dialyzed and fractionated by using a MonoS column to perform the purification.

An example of purification of the Sso7d protein obtained from *Sulfolobus solfataricus* is shown in the working examples mentioned hereinafter. It can also be purified from bacterial cells such as *Escherichia coli* cells in which the protein is highly produced thanks to a Sso7d protein gene introduced by a gene recombination technique.

The hybridization can be performed in the same manner as the usual nucleic acid hybridization, except that the hybridization is performed in the presence of a double-stranded DNA-binding protein. Specifically the hybridization can be performed as follows. First reducing agents such as dithiothreitol(DTT) and 2-mercaptoethanol, bovine serum albumin(BSA) and skim milk which prevent protein from non-specific biding to vessel and stabilize protein are added, and further protein accessory factors such as magnesium chloride(MgCl₂), salt-condensation regulators such as sodium chloride(NaCl) and potassium chloride(KCl) and so forth are added as required into buffer such as Tris buffer, phosphate buffer, citric acid byffer, TES buffer, HEPES buffer or the like. The double-stranded DNA biding protein is then added to the solution. In this hybridization solution, the aforementioned oligonucleotide-immobilized nylon membrane (DNA (macro)array) and a labeled sample DNA are hybridized preferably for 1-20 minutes within a range of 40-120°C. When Sso7d is used as the double-staranded DNA binding protein, to a Tris buffer, 0.1-100 mM of DTT, 0.1-100 mM of MgCl₂ and 1-100 µg/µl of BSA (all of the concentrations are final concentrations) are added, and the Sso7d protein is added to the solution within a range of 0.001-10%. In this hybridization solution, the oligonucleotide-immobilized nylon membrane (DNA (macro)array) and a labeled sample DNA are hybridized preferably for 1-15 minutes within a range of 40-70°C.

After the hybridization reaction, the membrane was washed with buffer such as Tris buffer, phosphate buffer, citric acid byffer, TES buffer, HEPES buffer or the like for 1-10 minutes within a range of 10-50°C, and then dried. In this case, adding a small amount of surfactant such as sodium dodecyl sulfate (SDS) is preferable because it can prevent from non-specific binding and step down backgraound. When Sso7d is used as the double-staranded DNA binding protein, after the hybridization reaction, it is preferable that the membrane should be washed with citric acid buffer such as SSC, more preferably buffer added with SDS within a range of 0.001-0.05% as required to SSC, for 3-10 minuites within a range of 10-40 °C, and then air-dried. As for the hybridization signal, radiation dose or the like of each dot on the dried nylon membrane can be measured by autoradiography etc. to calculate the hybridization strength.

Detection of hybridization can be measured by a method suitable for each of various labeling methods. In the gene expression monitoring method, it is preferable to use fluorescent labeling because this method enables simultaneous detection of the expression strength for a plurality of sample nucleic acids by labeling them with a plurality of fluorescent dyes each having a different detection wavelength.

Examples of the application of the nucleotide sequence analysis according to the present invention include: DNA nucleotide sequence determination [Genomics, Vol. 4, p. 114-128 (1989)], diagnosis of infectious and genetic diseases etc. [J.G. Hacia et al., Nature Genetics, 14, 441-447 (1996)], mapping of giant genome DNA [BioTechniques, vol.17, No.2, p.328-336 (1994)], single-nucleotidepolymorphisms (SNPs) [Wang et al. Science, Vol. 280, p. 1077-1082 (1998)], the amplification of genes or analysis of deleted regions by CGH (comparative genomic hybridization) method [D. Pinkel et al. Nat. Genet. Vol. 20, p. 207-211 (1998)], gene expression monitoring [M. Shena et al., Proc. Natl. Acad. Sci. USA, Vol. 93, pp.10614-10619 (1996)] and so forth.

As a diagnostic method for infectious diseases, there can be mentioned, for example, a method of detecting presence of a causative factor by extracting DNA from subject's blood etc., preparing a DNA probe based on a sequence specific to each of various pathogens [A. Troesch et al., J. Clin. Microbiol., Vol.37, p.49-55(1999)], and performing hybridization reaction according to the present invention to perform gene analysis for the extracted DNA.

As a diagnostic method for genetic diseases, there can be mentioned, for example, a method of detecting presence or absence of a mutation in a gene by preparing an oligonucleotide based on a sequence specific to a causative gene of genetic disease [M. J. Kozal et al., Nature Medicine, Vol.2, p.753-759(1996)], and performing hybridization of the oligonucleotide with chromosomal DNA obtained from a subject according to the present invention to perform gene analysis.

The giant genome DNA mapping is an essential technique for the genome DNA analysis project and so forth. By performing hybridization of many DNA probes prepared by the method of the present invention with genes of a genome bank, the location of each clone on the genome can be determined.

Further, a test kit for using to performe the aforementioned gene analysis can be prepared by using a double-stranded DNA-binding protein. Such a test kit is constituted by components similar to those of ordinary test kits for gene analysis utilizing hybridization except for the use of the double-stranded DNA-binding protein. That is, the test kit of the present invention comprises at least a double-stranded DNA-binding protein and, as optional components, washing solution, diluent, hybridization solution and so forth.

### EXAMPLES

The present invention will be explained more specifically with reference to the following examples. However, the present invention is no way limited by these examples.

### Example 1

### (A) Synthesis of probe DNA and sample DNA

The oligonucleotides shown in Table 1 were synthesized by using a DNA synthesizer (apparatus name: Expedite 8909) manufactured by PerSeptive Biosystems.

Immobilization of the oligonucleotides may be facilitated by modifying their 5' or 3' ends with 5' amino-modified C6 (produced by Glen Research) or the like. The oligonucleotide numbers used below corresponds to SEQ ID NOS in SEQUENCE LISTING.

The oligonucleotide (3) is a sample DNA to be analyzed, the oligonucleotide (1) is a DNA probe completely complementary to the oligonucleotide (3) (completely matched), and the oligonucleotide (2) is a DNA probe complementary to the oligonucleotide (3), but containing one nucleotide mismatch in the internal region.

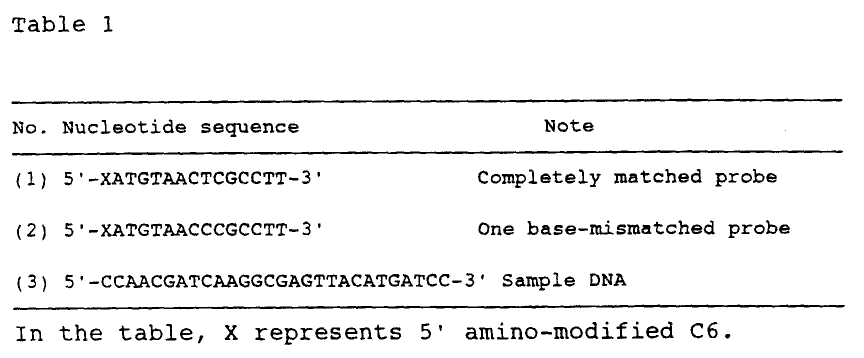

The synthesis of the aforementioned oligonucleotides and the sample DNA were performed in accordance with a standard protocol (Nucleic Acid Synthesis System User's Guide, PerSeptive Biosystems) by using a cycle in which the trityl groups were not removed, which were the protective groups of the 5' ends. The synthesized DNAs were purified by using Poros Oligo R3 (produced by PerSeptive Biosystems).

The oligonucleotides (1) and (2), and the sample DNA (3) were concentrated to dryness, and then suspended in a 0.5 M sodium hydrogencarbonate buffer (pH 8.4) for (1) and (2), or TE buffer for (3), and they are quantified based on absorbance at 260 nm and adjusted to 1 nmol/µl.

### (B) Immobilization of probe DNA (preparation of DNA array)

Immobilization of the oligonucleotides on a substrate was attained by bonding amino groups of the amino-modified oligonucleotides to a nylon membrane containing anionic carboxyl groups on its surface at a high density through amide bonds, as described below.

A Biodyne C (trade mark, produced by Pall) membrane was rinsed with 0.1 N HCl to acidify it, and immersed in 20% EDC (1-ethyl-3-dimethylaminopropylcarbodiimide hydrochloride) at room temperature for 15-30 minutes. The membrane was lightly rinsed with deionized water and 0.5 M sodium hydrogencarbonate buffer (pH 8.4), then mounted on a dot blot apparatus (produced by Bio-Rad) and allowed to react with the amino-modified oligonucleotide (1) or (2), which was suspended in a 0.5 M sodium hydrogencarbonate buffer (pH 8.4), at room temperature for 15 minutes.

The membrane was washed with TBS (Tris-buffered saline)/0.1% Tween-20, then treated with 0.1 N NaOH for 10 minutes, lightly rinsed with deionized water, and air-dried.

### (C) Labeling of sample DNA

As for the labeling of the sample DNA, the 5' end was radioactively-labeled with [γ-³²P] ATP. The reaction was performed by using a DNA 5' end labeling kit (MEGALABEL, produced by Takara Shuzo).

### (D) Purification of Sso7d protein

Cultivation was performed at 75°C under an aerobic condition in a medium (1 g of yeast extract, 2.3 g of (NH₄)₂SO₄, 0.08 g of CaCl₂ 2H₂O, 0.25 g of MgSO₄ 7H₂O, 0.28 g of KH₂PO₄, 0.3 g of Na₂SiO₃ 9H₂O, 0.03 g of Na₂MoO₄ 2H₂O, 0.02 g of FeSO₄ 7H₂O in 1 L of distilled water, adjusted to pH 3.0 with sulfuric acid) added with 10 g/L of saccharose, using a membrane fermenter. The cells were given with heat shock at 88°C for 90 minutes, and harvested by centrifugation.

The cells (100 g) were dissolved in Buffer A (10 mM Tris buffer, pH 8.8, 20 mM NaCl, 10% glycerol), and then disrupted with a French press. The disrupted cell suspension was centrifuged, and the obtained supernatant was dialyzed against Buffer A. Then, the dialysate was applied to a MonoQ column (produced by Pharmacia) and equilibrated with Buffer A. The Sso7d protein was obtained in the outflow fraction. This fraction was concentrated with Amicon, applied to a Superose 6 column and equilibrated with Buffer B (30 mM Tris-HCl buffer, pH 7.4, 200 mM NaCl). The fractions containing Sso7d were collected and dialyzed against Buffer C (50 mM potassium phosphate, pH 6.0, 50 mM NaCl), and then applied to a MonoS column (produced by Pharmacia). The column was equilibrated with Buffer C and eluted with a gradient using Buffer D (50 mM potassium phosphate, pH 8.0, 1 M NaCl) to obtain the Sso7d protein at a 25% concentration of Buffer D.

### (E) Hybridization reaction

### (a) Hybridization strength

70 µg/ml of Sso7d protein was added to Buffer E (20 mM Tris buffer, pH 7.5, 2 mM DTT, 5 mM MgCl₂, 10 µg/µl of BSA), and the aforementioned oligonucleotide-immobilized nylon membrane and the radioactively-labeled sample DNA were allowed to hybridize at 60°C for 3 minutes in the buffer. As a control, hybridization reaction was also performed in the same manner without adding the Sso7d protein.

After the hybridization reaction, the membrane was washed with 1 x SSC/0.03% SDS buffer at 30°C for 5 minutes and air-dried. The hybridization signal was evaluated by measuring radiation dose of each dot on the air-dried nylon membrane by autoradiography to calculate hybridization signal strength. The results are shown in Table 2. The hybridization signal strength is represented with relative values based on the hybridization signal strength of the probe oligonucleotide (1) when Sso7d was added, which is taken as 100.

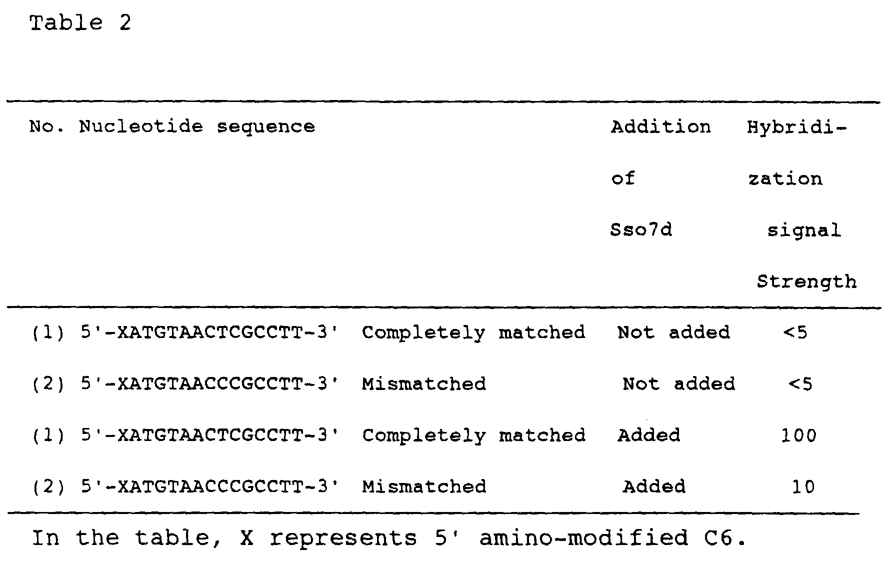

In the hybridization systems not added with Sso7d, hybridization between the probe DNA immobilized on the substrate (on the DNA array) and the sample DNA was not substantially detected for both of the completely matched probe (1) and the mismatched probe (2).

On the other hand, when the Sso7d protein was added to the hybridization systems, only the completely matched probe hybridized with the sample DNA on the DNA array. In comparison with the hybridization signal strength of the completely matched probe, the hybridization signal strength of the mismatch probe was considerably weaker, and these were clearly distinguishable.

### (b) Change of hybridization signal strength with change of Sso7d protein concentration

The Sso7d protein was added to Buffer E at a concentration of 70 µg/ml, 50 µg/ml, 25 µg/ml or 10 µg/ml, and the nylon membrane on which the completely matched probe in Table 1, the probe oligonucleotide (1), was immobilized, and the radioactively-labeled sample DNA were hybridized at 60°C for 3 minutes in each solution.

After the hybridization reaction, the membrane was washed with a solution of 1 x SSC/0.03% SDS buffer at 30°C for 5 minutes, and then air-dried. The hybridization signal was evaluated by measuring radiation dose of each dot on the air-dried nylon membrane by autoradiography to calculate hybridization signal strength. The results are shown in Table 3.

In the table, the hybridization signal strength is represented with relative values based on the hybridization signal strength obtained when Sso7d was added at a concentration of 70 µg/ml, which is taken as 100.

**Table 3**

| No. | Addition of Sso7d | Hybridization signal Strength |
|---|---|---|
| (1) | 70 µg/ml | 100 |
| (2) | 50 µg/ml | 100 |
| (3) | 25 µg/ml | 40 |
| (4) | 10 µg/ml | 10 |

The completely matched probe strongly hybridized to the sample DNA on the DNA array at a Sso7d protein concentration of 50 µg/ml or more. However, at the concentrations of 25 µg/ml and 10 µg/ml, the hybridization signal strength was weak. These results show that the hybridization signal sensitivity was increased by addition of Sso7d.

### (c) Change of hybridization signal strength with hybridization reaction time

A nylon membrane on which the completely matched probe, the oligonucleotide (1), was immobilized, and a radioactively-labeled sample DNA were allowed to hybridize at 60°C under the same condition as the above (E) (a), except that the hybridization reaction time was changed. The hybridization signal strength was measured at 3, 10 and 30 minutes after the start of the hybridization.

The hybridization signal strength did not vary with the reaction time. The hybridization had been almost fully completed within 3 minutes of reaction time, thus showing that the hybridization reaction speed was increased with the addition of the Sso7d protein.

### Example 2

### (A) Synthesis of probe DNA and sample DNA

The oligonucleotides shown in Table 4 were synthesized by using a DNA synthesizer (apparatus name: Expedite 8909) manufactured by PerSeptive Biosystems. Immobilization of the oligonucleotides may be facilitated by modifying their 5' or 3' ends with 3' amino-modified C6 (produced by Glen Research) or the like. As for the end of the sample DNA, its 5' end may be labeled with fluorescence using Cy5 Amidite (produced by Amersham Pharmacia Biotech). The oligonucleotide numbers used below corresponds to SEQ ID NOS in SEQUENCE LISTING.

The oligonucleotide (8) is a sample DNA to be analyzed, the oligonucleotides (4) and (6) are DNA probes completely complementary to the oligonucleotide (8) (completely matched), and the oligonucleotides (5) and (7) are DNA probes complementary to the oligonucleotide (8), but containing one nucleotide mismatch in the intermediary region.

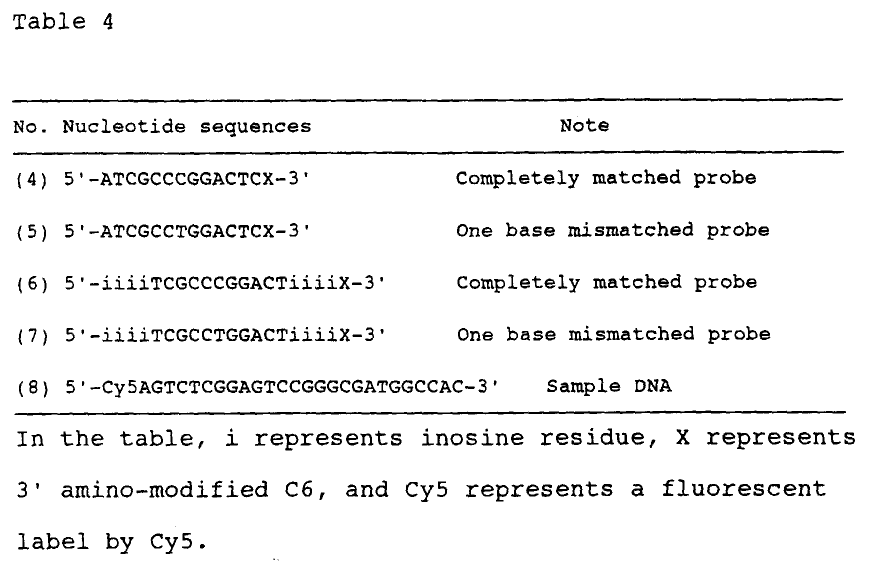

The syntheses of the aforementioned oligonucleotides and the sample DNA were performed in accordance with a standard protocol (Nucleic Acid Synthesis System User's Guide, PerSeptive Biosystems) by using a cycle in which the trityl groups were not removed, which were the protective groups of the 5' ends. The synthesized DNA were purified by using Poros Oligo R3 (produced by PerSeptive Biosystems).

The oligonucleotides (4) to (8) and the sample DNA were concentrated to dryness, and then suspended in a 0.5 M sodium hydrogencarbonate buffer (pH 8.4) for (4) to (7), or TE buffer for (8), and they are quantified based on absorbance at 260 nm and adjusted to 100 pmol/µl.

### (B) Immobilization of probe DNA (preparation of DNA microarray or DNA chip)

Immobilization of the oligonucleotides was performed as follows. First, solutions of the oligonucleotide probe nucleic acids (4) to (7) were each spotted onto a Silylateds Slide of TeleChem (slide glass having aldehyde groups on its surface) using a GTMASS Stamping apparatus produced by Nippon Laser & Electronics Lab. Then, according to the protocol of TeleChem, the nucleic acid was bonded on the slide glass through terminal covalent bonds (a Schiff base was formed by amino group and aldehyde group) as follows. First, the slide glass was mounted on a slide rack, and washed twice with 0.2% SDS at 25°C for 2 minutes with sufficient stirring in a beaker. Then, it was washed twice with sterilized water at 25°C for 2 minutes with sufficient stirring, and further treated with sterilized water at 98°C for 2 minutes. The slide glass was air-dried at room temperature for 5 minutes, then transferred into a sodium hydrogenborate solution [prepared by dissolving 1 g of NaBH₄ in 300 ml of PBS buffer (prepared by dissolving 8 g of sodium chloride, 0.2 g of potassium chloride, 1.44 g of disodium hydrogenphosphate, and 0.24 g of potassium dihydrogenphosphate in deionized water, adjusting it to pH 7.4 with hydrochloric acid, and filling it up to 1000 ml) and 100 ml of ethanol], treated in the solution at 25°C for 5 minutes, washed three times with 0.2% SDS at 25°C (room temperature) for 1 minute, finally washed with sterilized water at 25°C for 1 minute, and air-dried.

### (C) Hybridization reaction

70 µg/ml of the Sso7d protein and 100 pmol/ml of the sample DNA (8) were added to Buffer E (20 mM Tris buffer, pH 7.5, 2 mM DTT, 5 mM MgCl₂, 10 µg/ml of BSA), and the aforementioned oligonucleotide-immobilized slide glass (DNA microarray) and the fluorescently labeled sample DNA were allowed to hybridize at 60°C for 6 minutes in the solution. As a control, hybridization reaction was also performed in the same manner without adding the Sso7d protein.

After the hybridization reaction, the membrane was washed with 1 x SSC/0.03% SDS buffer at 25°C for 5 minutes, rinsed with 0.2 x SSC, and then further rinsed with 0.5 x SSC. After the washing solution was removed by centrifugation, the slide glass was air-dried.

The hybridization signal was evaluated by measuring amount of fluorescent dye of each spot on the air-dried slide glass by using ScanArray 3000 produced by Genaral Scanning to calculate the hybridization signal strength. The results are shown in Table 5. The results are represented with relative values based on the hybridization signal strength of the probe DNA (4) obtained with the addition of Sso7d, which is taken as 100.

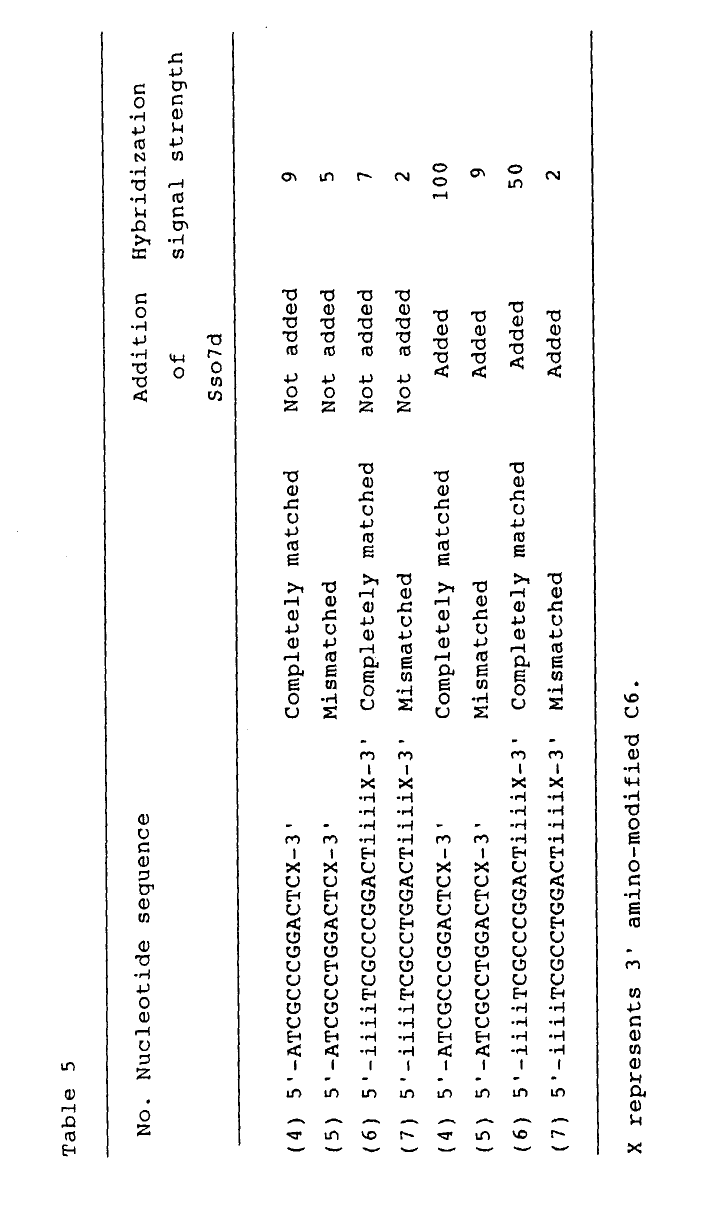

In the hybridization systems not added with Sso7d, hybridization between the probe DNA immobilized on the substrate (on the DNA array) and the sample DNA was not substantially detected under the aforementioned conditions.

On the other hand, when the Sso7d protein was added to the hybridization systems, only the completely matched probes hybridized with the sample DNA on the DNA array. In comparison with the hybridization signal strength of the completely matched probes, the hybridization signal strength of the mismatched probes was considerably weaker, and these were clearly distinguishable.

According to the present invention, gene analysis by hybridization utilizing a probe nucleic acid can be quickly performed with high precision and high sensitivity.

Having thus described the present invention, it will be obvious that several aspects of the invention may be modified in various ways. Such variations are not to be regarded as departures from the spirit and scope of the invention, and all such modifications would be obvious to those skilled in the arts, and are intended to be included within the scope of the following claims.

## Claims

1. A method for gene analysis comprising the step of detecting hybridization between a probe nucleic acid and a sample nucleic acid containing a target sequence that has a sequence complementary to that of the probe nucleic acid, wherein either the probe nucleic acid or the sample nucleic acid is immobilized on a substrate, at least one of the probe nucleic acid and the sample nucleic acid is DNA, and the hybridization is caused in the presence of a double-stranded DNA-binding protein.

2. The method of claim 1, wherein the sample nucleic acid is DNA.

3. The method of claim 1 or 2, wherein the double-stranded DNA-binding protein is derived from a hyperthermophilic bacterium.

4. The method of claim 3, wherein the double-stranded DNA-binding protein is derived from an archaebacterium.

5. The method of claim 4, wherein the double-stranded DNA-binding protein is derived from a bacterium belonging to the genu*s Sulfolobus*.

6. The method of claim 5, wherein the double-stranded DNA-binding protein is derived from *Sulfolobus solfataricus*.

7. The method of claim 6, wherein the double-stranded DNA-binding protein is the Sso7d protein derived from *Sulfolobus solfataricus*.

8. The method of any one of claims 1 to 7, wherein the double-stranded DNA-binding protein is a protein having homology of 75% or more to the protein represented by the amino acid sequence of SEQ ID NO: 9.

9. The method of any one of claims 1 to 8, wherein the sample nucleic acid is labeled.

10. The method of any one of claims 1 to 9, wherein amount of the sample nucleic acid containing the target sequence is analyzed based on intensity of hybridization signal.

11. The method of any one of claims 1 to 9, wherein detecting hybridization is performed by using a plurality of probe nucleic acids and then polymorphism in the target sequence is detected based on the result of detection of hybridization.

12. The method of any one of claims 1 to 9, wherein detecting hybridization is performed by using a plurality of probe nucleic acids and then the nucleotide sequence of the sample nucleic acid is determined based on the result of detection of hybridization.

13. A test kit for the detection of hybridization between a probe nucleic acid and a sample nucleic acid containing a target sequence that has a sequence complementary to that of the probe nucleic acid, which comprises at least a double-stranded DNA-binding protein.
